# EUROPEAN PATENT APPLICATION

(11) **EP 2 397 504 A1**
(43) Date of publication of application: **21.12.2011**
(21) Application number: 10741213.2
(22) Date of filing: 09.02.2010
(51) Int. Cl.: C08F 20/30, C07C 69/76, C09D 4/02, C09D 7/12, C09D 175/14, G02B 1/04, G02B 1/10

(54) **(METH)ACRYLATE COMPOUND CONTAINING AROMATIC GROUP**

(30) Priority: 13.02.2009 JP 2009030864
(71) Applicant: Showa Denko K.K., Tokyo 105-8518 (JP)
(72) Inventor: URAKAWA, Yoshifumi, Tokyo 105-8518 (JP); YAMAKI, Shigeru, Tokyo 105-8518 (JP); ISHII, Nobuaki, Tokyo 105-8518 (JP); MAEDA, Yoshihiko, Tokyo 105-8518 (JP)
(74) Representative: Strehl Schübel-Hopf & Partner
(86) International application number: PCT/JP2010/051841
(87) International publication number: WO 2010/092936

(57) **Abstract**

Disclosed is a (meth)acrylate compound which can provide a cured article having a high refractive index and excellent transparency and heat resistance, and which has superior handling characteristics; further disclosed are a curable composition containing the (meth)acrylate compound, and a cured article thereof. The (meth) acrylate compound contains an aromatic group represented by the following general formula (1), the curable composition comprising the aromatic-group containing (meth) acrylate compound, and the cured article thereof. (In the formula, R₁, R₂, R₃ and R₄ are each independently a hydrogen atom or a methyl group; X is an organic group having an aromatic group and 6 to 30 carbon atoms; and a and b are each independently an integer of 0 to 3.)

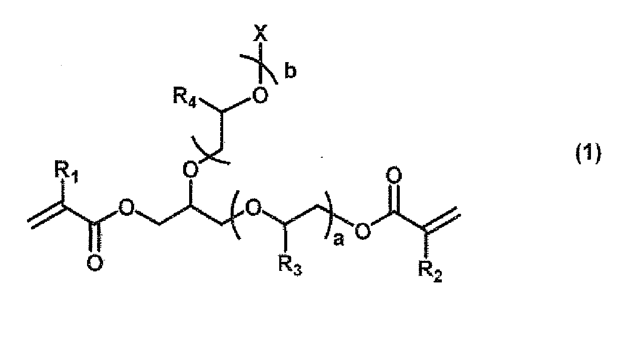

## Description

### Technical Field

The present invention relates to an aromatic group-containing (meth)acrylate compound which is suitable as a material to produce an optical component, such as a transparent material, in particular a lens; a curable composition comprising the compound; and a cured article obtained by curing the composition. More specifically, the present invention relates to a cured article with a high refractive index and superior heat resistance which is obtained by curing a curable composition excellent in handling characteristics comprising a low-viscosity aromatic group-containing (meth)acrylate compound.

With the recent development of optical industry represented by optical equipment, optical communication and displays, a material with superior optical performance has been demanded. As such a material, for example, an optical lens, a optical disk substrate, a plastic substrate for a liquid crystal display element, a substrate for a color filter, a plastic substrate for organic EL display element, a solar cell substrate, a touch panel, an optical element, an optical waveguide, a LED sealant, and the like can be mentioned. The demand has been high particularly for materials for the optical lens, optical element and optical waveguide.

In general, an inorganic glass has been often used as materials for substrate for a liquid crystal display element, substrate for a color filter, substrate for an organic EL display element, solar cell substrate, touch panel, and the like. But, the glass plate has problems such as its likelihood to break, its inability to bend, a large specific gravity not suitable for weight-reducing. Thus, many attempts have been made recently to replace the glass plate with a plastic material.

Meanwhile, as materials for the optical lens, optical element, optical waveguide and LED sealant, there has been recent demand for plastic materials excellent in heat resistance, such as those having a reflow resistance. On the other hand, the plastic material, if having a low refractive index, will have a thick edge, and impair its lightness, a feature of the plastic. In view of this,materials having a higher refractive index have been demanded.

For example, JP-A-H10-77321 (Patent Document 1) describes that an element obtained by curing, using an active energy ray, a resin composition containing an amorphous thermoplastic resin and bis (meth) acrylate capable of being cured by the active energy ray can replace the glass substrate and can be suitably used for an optical lens, optical disk substrate, plastic liquid crystal substrate, and the like. However, there is concern that the transparency is reduced as a result of the difference between the refractive indexes of the amorphous thermoplastic resin and of the resin obtained by cuing the bis(meth)acrylate using the active energy ray.

Further, JP-A-H04-325508 (Patent Document 2) indicates that the introduction of a fluorene skeleton into a (meth)acrylate compound results in improved refractive index and heat resistance. However, the compound having a fluorene skeleton has an extremely high viscosity and thus has poor handling characteristics, failing to satisfy the recent demand for a compound having a lower viscosity.

JP-A-2005-272773 (Patent Document 3), describing a cured article obtained by curing a curable composition containing a compound having a biphenyl skeleton, indicates that such a composition has a low viscosity and can provide a cured article having a high refractive index. However, the document is silent with regard to heat resistance: Considering that the compound having a biphenyl skeleton is a monofunctional monomer, and the heat resistance is still a problem.

### Citation List

### Patent Documents

Patent Document 1: JP-A-H10-77321
Patent Document 2: JP-A-H04-325508
Patent Document 3: JP-A-2005-272773

### Summary of the Invention

### Problem to be Solved by the Invention

It is an object of the present invention to provide an aromatic group-containing (meth) acrylate compound with superior handling characteristics which can provide a cured article having a high refractive index and excellent transparency and heat resistance. It is another object of the present invention to provide a curable composition comprising the aromatic group-containing (meth)acrylate compound, and an cured article obtained using the curable composition.

### Means for Solving the Problem

The present inventors have earnestly studied to solve the above problems, and have found out that the above problems can be solved by an aromatic group-containing (meth) acrylate compound having a specific structure and a curable composition comprising the compound. Herein, the (meth)acrylate of the aromatic group-containing (meth)acrylate compound refers to an acrylate and/or a methacrylate. Hereinafter, the same meaning applies to the other (meth)acrylate compounds.

That is, the present invention relates to the following.
[1] An aromatic group-containing (meth) acrylate compound represented by the following general formula (1): 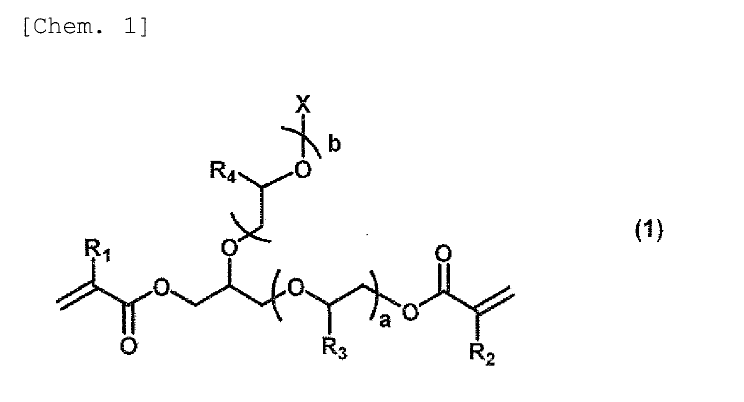 In the formula, R₁, R₂, R₃ and R₄ are each independently a hydrogen atom or a methyl group; X is an organic group having an aromatic group and 6 to 30 carbon atoms; and a and b are each independently an integer of 0 to 3.
[2] An aromatic group-containing (meth) acrylate compound represented by the following general formula (2): 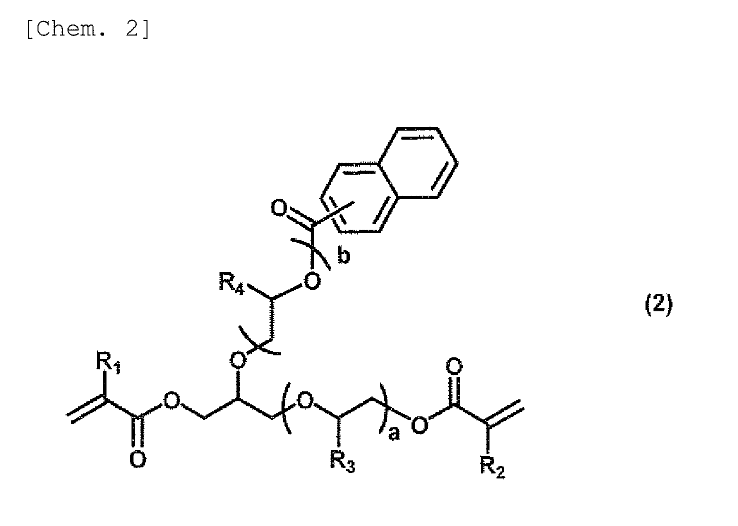 In the formula, R₁, R₂, R₃ and R₄ are each independently a hydrogen atom or a methyl group; and a and b are each independently an integer of 0 to 3.
[3] An aromatic group-containing (meth) acrylate compound represented by the following general formula (3): 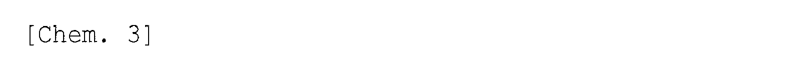 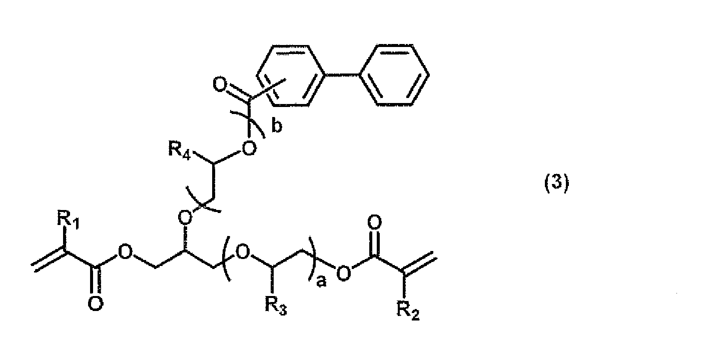 In the formula, R₁, R₂, R₃ and R₄ are each independently a hydrogen atom or a methyl group; and a and b are each independently an integer of 0 to 3.
[4] A curable composition comprising the aromatic group-containing (meth) acrylate compound as described in any one of [1] to [3] and a polymerization initiator.
[5] The curable composition as described in [4], which comprises 0.1 to 5 parts by mass of the polymerization initiator based on 100 parts by mass of the aromatic group-containing (meth)acrylate compound.
[6] The curable composition as described in [4], which further comprises a urethane oligomer and/or a reactive monomer.
[7] The curable composition as described in [6], which comprises 0.1 to 5 parts by mass of the polymerization initiator based on 100 parts by mass of the total of the aromatic group-containing (meth)acrylate compound and the urethane oligomer and/or the reactive monomer.
[8] The curable composition as described in any one of [4] to [7], which has a viscosity at 25°C of from 10 mPa·s to 10,000 mPa·s.
[9] A cured article which is obtained by curing the curable composition as described in any one of [4] to [8] and which has a refractive index of from 1.55 to 1.65.
[10] A cured article which is obtained by curing the curable composition as described in any one of [4] to [8] and which has a glass transition temperature of from 80°C to 200°C.
[11] A coating material which is obtained by curing the curable composition as described in any one of [4] to [8].
[12] An optical material which is obtained by curing the curable composition as described in any one of [4] to [8].
[13] A lens which is obtained by curing the curable composition as described in any one of [4] to [8].

### Effect of the Invention

According to the present invention, there is provided an aromatic group-containing (meth)acrylate compound with superior handling characteristics which can provide a cured article having a high refractive index and excellent transparency and heat resistance. There are also provided a curable composition comprising the compound and a cured article thereof.

According to the present invention, there can be provided a cured article which can be suitably used for an optical lens, a optical disk substrate, a plastic substrate for a liquid crystal display element, a substrate for a color filter, a plastic substrate for an organic EL display element, a solar cell substrate, a touch panel, an optical element, an optical waveguide, a LED sealant, and the like.

### Brief Description of the Drawings

[Fig. 1]

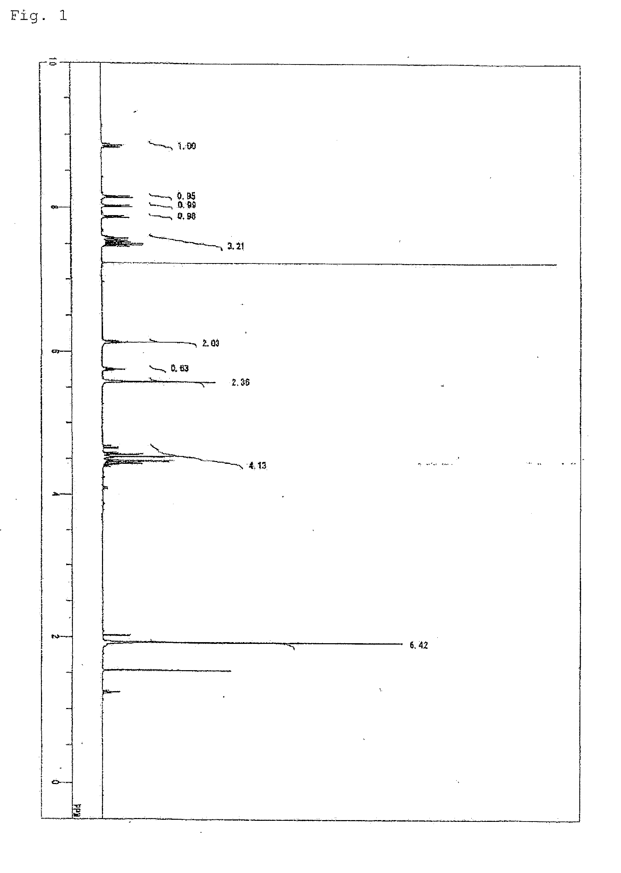

   Fig. 1 shows a ¹H-NMR chart for an aromatic group-containing methacrylate compound (A-1) synthesized in Example 1.
[Fig. 2]

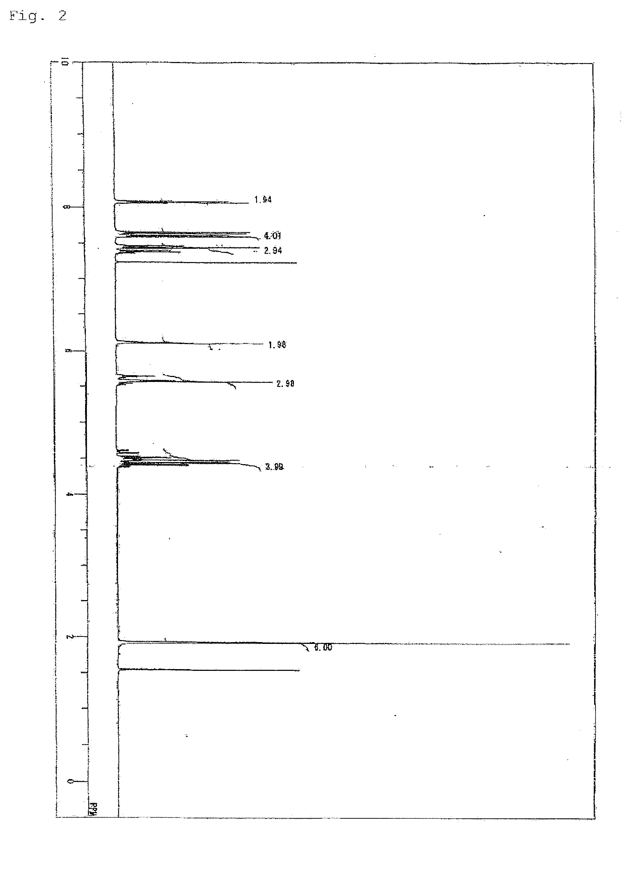

   Fig. 2 shows a ¹H-NMR chart for an aromatic group-containing methacrylate compound (A-5) synthesized in Example 5.

### Embodiments for Carrying Out the Invention

Hereinafter, embodiments for carrying out the present invention will be described in detail.

### Aromatic Group-Containing (Meth)acrylate Compound

An aromatic group-containing (meth)acrylate compound represented by the following general formula (1) (hereinafter, also called an "aromatic group-containing (meth)acrylate (1)") comprises two ethylenically unsaturated bonds in a molecule.

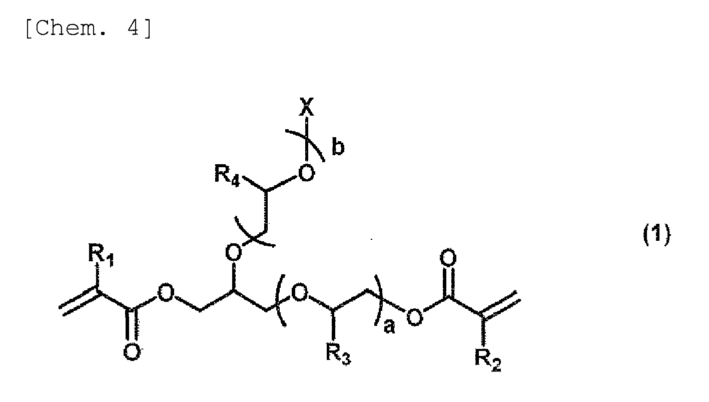

In the general formula (1), R₁, R₂, R₃ and R₄ are each independently a hydrogen atom or a methyl group; X is an organic group having an aromatic group and 6 to 30 carbon atoms; and a and b are each independently an integer of 0 to 3.

In the general formula (1), R₁, R₂, R₃ and R₄ are preferably methyl groups in view of improving the heat resistance and pencil hardness.
In the general formula (1), a and b are preferably each independently 0 or 1, more preferably 0 in view of improving the heat resistance and pencil hardness and also in view of the easy availability of a material.

In the general formula (1), the number of the carbon atoms of X is preferably 7 to 24, more preferably 7 to 19, still more preferably 7 to 15 in view of achieving a higher refractive index and a lower viscosity.

Specific examples of X include the following (a) to (h).

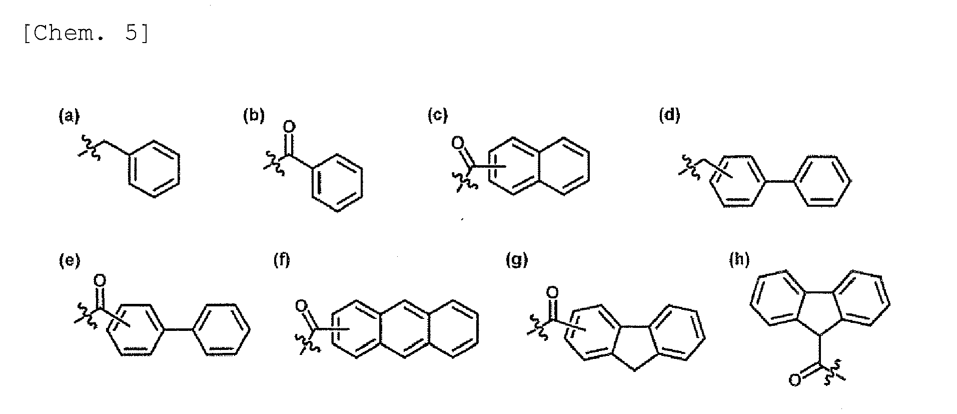

In the above formulae, a part with a wavy line denotes a bond with X in the compound represented by the general formula (1).
Among the above specific examples, those having a naphthoyl skeleton (c) and those having a phenylbenzoyl skeleton (e) are particularly preferred in view of the refractive index, viscosity and easy availability of a material.

That is to say, an aromatic group-containing (meth) acrylate compound represented by the following general formula (2) (hereinafter, also called an "aromatic group-containing (meth)acrylate (2)") is particularly preferred.

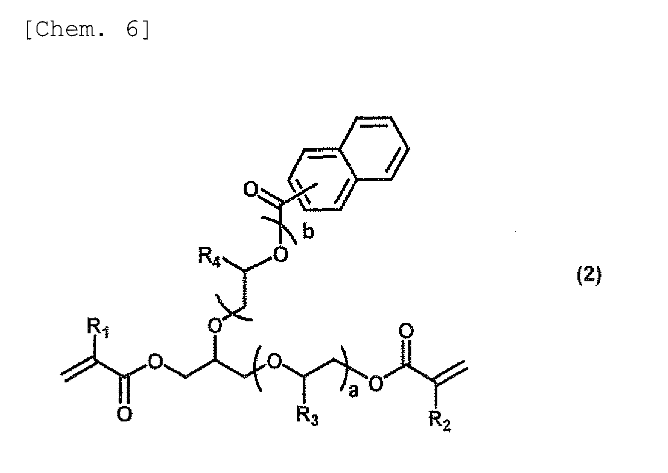

In the general formula (2), R₁, R₂, R₃ and R₄ are each independently a hydrogen atom or a methyl group; and a and b are each independently an integer of 0 to 3.
In the general formula (2), R₁, R₂, R₃ and R₄ are preferably methyl groups in view of improving the heat resistance and pencil hardness.

In the general formula (2), a and b are preferably each independently 0 or 1, more preferably 0 in view of improving the heat resistance and pencil hardness and also in view of the easy availability of a material.

Further, in the general formula (2), the bonding with naphthalene at α-position is more preferred in view of the handling characteristics.
That is to say, the following structure is particularly preferred.

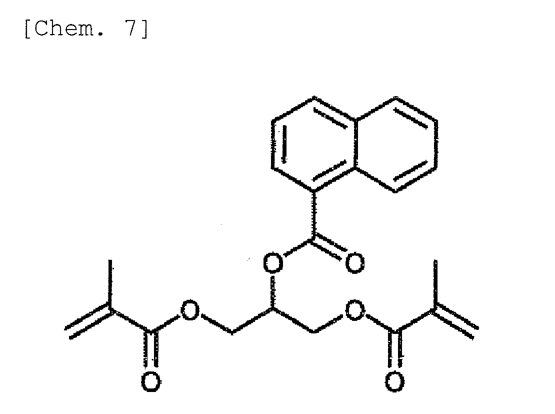

Further, as described above, a compound wherein X in the general formula (1) is a phenylbenzoyl skeleton, i.e., an aromatic group-containing (meth)acrylate compound represented by the following general formula (3) (hereinafter, also called an "aromatic group-containing (meth)acrylate (3)") is also particularly preferred.

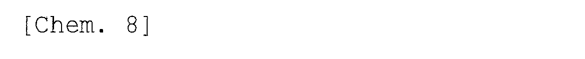

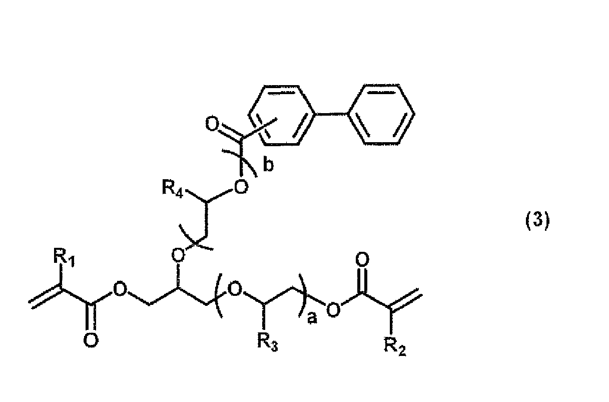

In the general formula (3), R₁, R₂, R₃ and R₄ are each independently a hydrogen atom or a methyl group; and a and b are each independently an integer of 0 to 3.
In the general formula (3), R₁, R₂, R₃ and R₄ are preferably methyl groups in view of improving the heat resistance and pencil hardness.

In the general formula (3), a and b are each independently 0 or 1, more preferably 0 in view of improving the heat resistance and pencil hardness and also in view of the easy availability of a material.

Further, in the general formula (3), the bonding of a carbonyl group at 4-position is preferred in view of the easy availability.
That is to say, the following structure is particularly preferred.

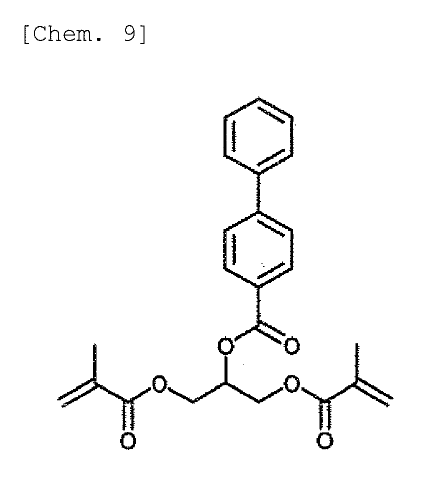

The viscosity at 25°C of the aromatic group-containing (meth) acrylate compound (1) is preferably from 10 mPa·s to 10,000 mPa·s, more preferably from 50 mPa·s to 5,000 mPa·s, most preferably from 100 mPa·s to 2,000 mPa·s.

When the viscosity of the aromatic group-containing (meth)acrylate compound (1) is less than 10 mPa·s or more than 10,000 mPa·s, the handling characteristics are poor and the workability is inferior and thus it is difficult to use.

In this regard, measurement conditions of the viscosity employs, as in a method indicated in the later-described Example, a B-type viscometer DV-II+Pro (manufactured by Brookfield Engineering Laboratories, Inc.), the rotor No. 42, and the rotation number of 1 to 7 rpm.

### Production Method of Aromatic Group-Containing (Meth)acrylate Compound (1)

The aromatic group-containing (meth)acrylate compound (1) can be synthesized by reacting a (meth)acrylate compound having a hydroxyl group represented by the following general formula (4) (hereinafter, also called a "(meth)acrylate compound (4)") with a compound having the same organic group as X in the general formula (1) and a substituent capable of being eliminated (hereinafter also called a "substituent Y") (hereinafter, the compound is also called a "compound (5)").

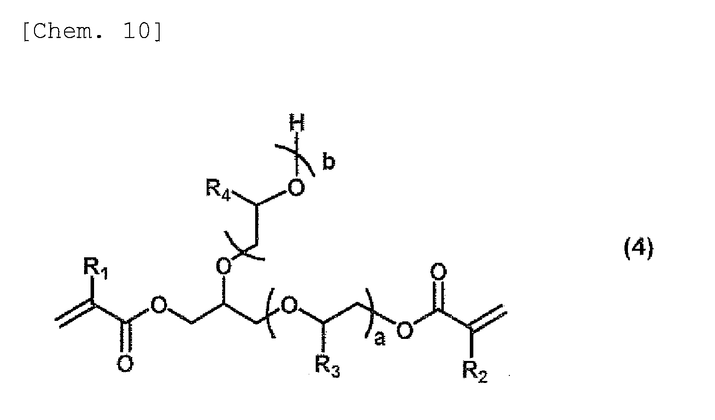

In the general formula (4), R₁, R₂, R₃ and R₄ are each independently a hydrogen atom or a methyl group; and a and b are each independently an integer of 0 to 3.
Examples of the (meth)acrylate compound (4) include glycerol dimethacrylate, glycerol acrylate methacrylate, ethylene oxide modified glycerol dimethacrylate, ethylene oxide modified glycerol acrylate methacrylate, propylene oxide modified glycerol dimethacrylate and propylene oxide modified glycerol acrylate methacrtlate.

In the compound (5), the substituent Y is any of those substituents which react with the hydroxyl group and is capable of being eliminated, with examples thereof including a halogen atom and an alkoxy group. In view of the reactivity and the easy availability, a chlorine atom, a bromine atom, a methoxy group and an ethoxy group are particularly preferred.
Examples of the compound (5) include benzyl chloride, benzyl bromide, benzyl chloromethyl ether, benzyl chloromethyl sulfide, 4-benzyloxyphenylacetyl chloride, benzoyl chloride, benzoyl bromide, methyl 2-benzoylbenzoate, benzoyl fluoride, methyl benzoylformate, ethyl benzoylformate, methyl benzoylpropionate, biphenyl chloride, 4,4'-biphenyl dicarbonyl chloride, 4,4'-bipheyl dimethyl dicarboxylate, 4,4'-bipheyl diethyl dicarboxylate, 4-phenylbenzoyl chloride, 4-phenylbenzyl bromide, 2-anthracene carboxylic acid, 9-anthracene carboxylic acid, 1,8-anthracenedicarboxylic acid dimethyl ester, anthraquinone-2-carbonyl chloride, 9-fluorene acetic acid, 9-fluorene carboxylic acid, 9-fluorenone-2-carboxylic acid, 9-fluorenylmethyl chloroformate, 2,2',4'-trichloroacetophenone, 2,3',4'-trichloroacetophenone, 2,4,6-trichlorobenzoyl chloride, triphenyl acetic acid, triphenyl methyl chloride, triphenyl methyl bromide, methyl diphenylacetate, diphenyl acetyl chloride, 1-naphthoyl chloride, and 2-naphthoyl chloride.

Regarding the molar ratio for the reaction between the (meth)acrylate compound (4) and the compound (5), the amount by mole of the hydroxyl group in the (meth)acrylate compound (4) : the amount by mole of the eliminating group in the compound (5) is preferably 1:1 to 1:2.

In the reaction between the (meth) acrylate compound (4) and the compound (5), a base is preferably used. The use of a base can make the reaction considerably faster. Specific examples of the base include triethylamine, 1,4-diazabicyclo[2.2.2]octane, 2,6,7-trimethyl-1,4-diazabicyclo[2.2.2]octane, potassium tert-butanolate, sodium hydroxide and an ion exchange resin.

These bases may be used singly or in combination of two or more kinds. The addition amount of the base is preferably 1.0 to 2.0 equivalents, more preferably 1.0 to 1.5 equivalents, per hydroxyl group in the (meth)acrylate compound (4). When the addition amount is less than 1.0 equivalent, the reactivity may be reduced. On the other hand, when the addition amount is more than 2.0 equivalents, the reaction may be accompanied by a side reaction.

In the reaction between the (meth) acrylate compound (4) and the compound (5), the reaction temperature is preferably -10 to 100°C, more preferably 0 to 80°C, still more preferably 10 to 40°C.

In the reaction between the (meth) acrylate compound (4) and the compound (5), specific examples of a solvent used include cyclic ethers such as tetrahydrofran and dioxane; amides such as N,N-dimtehylformamide; aromatic hydrocarbons such as toluene and xylene; halogenated hydrocarbons such as methylene chloride and chloroform; and acetonitrile. Preferred are tetrahydrofran, toluene and dichloromethane.

The reaction made under the above conditions can provide the aromatic group-containing (meth)acrylate compound (1) at a good yield and purity with a side reaction suppressed. Further, the reaction made at a temperature near a room temperature can reduce the possibility of the polymerization between the aromatic group-containing (meth)acrylate compounds (1).

The aromatic group-containing (meth)acrylate compound (1) can be synthesized also by reacting the (meth) acrylate compound (4) with a compound having the same organic group as X in the general formula (1) and having a carboxyl group at an end (hereinafter, also called a "compound (6)").

The (meth)acrylate compound (4) is as described above. Examples of the compound (6) include benzoic acid, 1-naphthoic acid, 2-naphthoic acid, 4-phenylbenzoic acid, 2-phenylbenzoic acid, 1-anthracene carboxylic acid, 2-anthracene carboxylic acid, 9-anthracene carboxylic acid, and 9-fluorene carboxylic acid. Preferred are 1-naphthoic acid and 4-phenylbenzoic acid.

Regarding the molar ratio for the reaction between the (meth)acrylate compound (4) and the compound (6), the amount by mole of the hydroxyl group in the (meth)acrylate compound (4) : the amount by mole of the carboxylic acid in the compound (6) is preferably 1:1 to 1:1.2.

In the reaction between the (meth) acrylate compound (4) and the compound (6), a condensation agent is preferably used. The use of a condensation agent can activate a carboxylic acid and make the reaction considerably faster. Specific examples of the condensation agent include N,N'-dicyclohexylcarbodiimide, 1-ethyl-3-(3-dimethylaminopropyl)carbodiimide, 1,1'-carbonyldiimidazole, 2-chloro-1-methylpyridiniumiodine, methyl·3-methyl-2-fluoropyridinium·tosylate, methanesulfonyloxybenzotriazole, and 1-propylphosphonic acid cyclic anhydrides. Preferred is 1-ethyl-3-(3-dimethylaminopropyl)carbodiimide.

These condensation agents may be used singly or in combination of two or more kinds. The addition amount of the condensation agent is preferably 1.0 to 2.0 equivalents, more preferably 1.0 to 1.5 equivalents, per carboxyl group in the compound (6). When the amount is less than 1.0 equivalent, the reactivity may be reduced. On the other hand, when the addition amount is more than 2.0 equivalents, the reaction may be accompanied by a side reaction or make a post-treatment cumbersome.

In the reaction between the (meth) acrylate compound (4) and the compound (6), a tertiary amine can be added as a catalyst. The use of a tertiary amine can make the reaction considerably faster. Specific examples of the tertiary amine include pyridine, N,N-dimethyl-4-aminopyridine, triethylamine, N,N-diisopropylethylamine, and N,N-diethylaniline. Preferred is N,N-dimtehyl-4-aminopyridine.

In the reaction between the (meth) acrylate compound (4) and the compound (6), the reaction temperature is preferably -10 to 80°C, more preferably 0 to 60°C, still more preferably 10 to 40°C.

In the reaction between the (meth) acrylate compound (4) and the compound (6), specific examples of a solvent used include cyclic ethers such as tetrahydrofran and dioxane; amides such as N,N-dimethylformamide; aromatic hydrocarbons such as toluene and xylene; halogenated hydrocarbons such as dichloromethane and chloroform; and acetonitrile. Preferred are tetrahydrofran, toluene and dichloromethane.

The reaction made under the above conditions can provide the aromatic group-containing (meth)acrylate compound (1) at a good yield and purity with a side reaction suppressed. Further, the reaction made at a temperature near a room temperature can reduce the possibility of the polymerization between the aromatic group-containing (meth)acrylate compounds (1).

### Curable Composition

The curable composition of the present invention comprises at least the aromatic group-containing (meth)acrylate compound of the present invention and a polymerization initiator.

In the present invention, as the polymerization initiator, a photopolymerization initiator or a thermal polymerization initiator can be used. The photopolymerization initiator is preferred from the viewpoint that it is usable also for a substrate having a low heat resistance.

In the case of using the photopolymerization initiator, the application of an active energy ray such as ultraviolet or a visible light causes the polymerization reaction of the components contained in the curable composition, and thereby a cured article is obtained.

Specific examples of the photopolymerization initiator include 1-hydroxycyclohexyl phenylketone, 2,2'-dimethoxy-2-phenylacetophenone, xanthone, fluorene, fluorenone, benzaldehide, anthraquinone, triphenylamine, carbazole, 3-methylacetophenone, 4-chlorobenzophenone, 4,4'-dimethoxybenzophenone, 4,4'-diaminobenzophenone, Michler's ketone, benzoyl propyl ether, benzoin ethyl ether, benzyl dimethyl ketal, 1-(4-isopropylphenyl)-2-hydroxy-2-methylpropane-1-one, 2-hydroxy-2-methyl-1-phenylpropane-1-one, phenyl glyoxylic acid methyl ester, thioxanthone, diethyl thioxanthone, 2-isopropyl thioxanthone, 2-chlorothioxanthone, 2-methyl-1-[4-(methylthio)phenyl]-2-morpholinopropane-1-one, 2,4,6-trimethyl benzoyl diphenyl phosphine oxide, ethyl(2,4,6-trimethylbenzoyl)phenyl phosphinate, 2-benzyl-2-dimethylamino-1-(4-morpholinophenyl)butane-1-one, 1-[4-(2-hydroxyethoxy)-phenyl]-2-hydroxy-2-methylpropane-1-on e. These photopolymerization initiators may be used singly or in combination of two or more kinds.

In the case of using the thermal polymerization initiator, heating causes the polymerization reaction of the aromatic group-containing (meth)acrylate compound (1) thereby to obtain a cured article. Specific examples of the thermal polymerization initiator include azo compounds and organic peroxides. Examples of the azo compounds include 2,2'-azobis(isobutylonitrile), 2,2'-azobis(2,4-dimethylvaleronitrile), dimethyl 2,2'-azobis(2-methylpropionate), 4,4'-azobis(4-cyanovaleric acid), 2,2'-azobis(2-amidinopropane)2-hydrochloride, and 2,2'-azobis{2-methyl-N-[2-(1-hydroxybutyl)]-propionamide}. Specific examples of the organic peroxides include benzoylperoxide, lauroylperoxide, tert-butylperoxy-2-ethylhexanoate, tert-butylperoxyneodecanoate, and 1,1,3,3-tetramethylbutylperoxy-2-ethylhexanoate. These thermal polymerization initiators may be used singly or in combination of two or more kinds.

The amount used of the polymerization initiator is not particularly limited, but is 0.1 to 5 parts by mass, preferably 0.5 to 3 parts by mass, more preferably 0.5 to 1 part by mass, based on 100 parts by mass of the aromatic group-containing (meth)acrylate compound (1). Further, when the curable composition of the present invention comprises a later-mentioned radical reactive component, i.e., a urethane oligomer and/or a reactive monomer, the amount used of the polymerization initiator is usually 0.1 to 5 parts, preferably 0.5 to 3 parts by mass, more preferably 0.5 to 1 part by mass, based on 100 parts by mass of the total of the aromatic group-containing (meth)acrylate compound (1) and the radical reactive component. By using the polymerization initiator at an amount within the above range, the polymerization rate of the aromatic group-containing (meth)acrylate compound (1) becomes faster, and the curable composition is not influenced by polymerization inhibition such as oxygen, and moreover, the resulting cured film has a high strength and heat resistance and is unlikely to be colored.

The curable composition of the present invention may comprise another component, for example, may comprise 0.1 part by mass or less of a polymerization inhibitor based on 100 parts by mass of the curable composition. The polymerization inhibitor is used to prevent components contained in the curable composition from causing polymerization reaction while the composition is stored. Examples of the polymerization inhibitor include hydroquinone, hydroquinone monomethyl ether, benzoquinone, p-t-butylcatechol and 2,6-di-t-butyl-4-methylphenol.

In addition to the aromatic group-containing acrylate compound (1) and the polymerization initiator as described above, the curable composition of the present invention may further comprise, as a radical reactive component, a urethane oligomer and/or a reactive monomer. The inclusion of these makes it possible to control characteristics of the resultant cured article, such as reactivity, mechanical properties including hardness, elasticity and adhesion, and optical properties including transparency.

The urethane oligomer as described above is an oligomer having a urethane bond and an ethylenically unsaturated bond. For example, it is possible to use a polymer having a relatively small molecular weight obtained by bonding 2 to 20 urethane monomers, the urethane monomer being obtained by reacting a compound having an isocyanate group and a compound having a hydroxyl group. Specific examples thereof include Beamset 102, 502H, 505A-6, 510, 550B, 551B, 575, 575CB, EM-90 and EM92 (product name) manufactured by Arakawa Chemical Industries, Ltd.; PHOTOMER 6008 and 6210 (product name) manufactured by SAN NOPCO LIMITED; NK Oligo U-2PPA, U-4HA, U-6HA, U-15HA, UA-32P, U-324A, U-4H, U-6H, UA-160TM, UA-122P, UA-2235PE, UA-340P, UA-5201 and UA-512 (product name) manufactured by Shin-Nakamura Chemical, Co., Ltd.; ARONIX M-1100, M-1200, M-1210, M-1310, M-1600, M-1960 and M-5700 and ARONOXETANE OXT-101 (product names) manufactured by TOAGOSEI Co., Ltd. ; AH-600, AT606, UA-306H, and UF-8001 (product name) manufactured by KYOEISHA CHEMICAL Co., LTD. ; KAYARAD UX-2201, UX-2301, UX-3204, UX-3301, UX-4101, UX-6101 and UX-7101 (product name) manufactured by NIPPON KAYAKU Co., Ltd.; Shiko UV-1700B, UV-3000B, UV-6100B, UV-6300B, UV-7000, UV-7600B, UV-7605B, UV-2010B, UV-6630B, UV-7510B, UV-7461TE, UV-3310B, and UV-6640B (product name) manufactured by The Nippon Synthetic Chemical Industry Co., Ltd.; ART RESIN UN-1255, UN-5200, UN-7700, UN-333, UN-905, HDP-4T, HMP-2, UN-901T, UN-3320HA, UN-3320HB, UN-3320HC, UN-3320HS, H-61, HDP-M20, UN-5500, and UN-5507 (product name) manufactured by Negami Chemical Industrial Co., Ltd.; and Ebecryl 6700, 204, 205, 220, 254, 1259, 1290K, 1748, 2002, 2220, 4833, 4842, 4866, 5129, 6602, and 8301 (product name) manufactured by Daicel-UCB Co., Ltd.

These may be used singly or in combination of two or more kinds.
The above reactive monomer, also called a reactive diluent, is a monomer having an ethylenically unsaturated bond and may be a monofunctional monomer or a multifunctional monomer. Specific examples thereof include ethylenically unsaturated aromatic compounds, carboxyl group-containing unsaturated compounds, monofunctional (meth)acrylates, di(meth)acrylates, multifunctional (meth)acrylates, epoxy poly(meth)acrylates, urethane poly(meth)acrylates, and polyester poly(meth)acrylates. Hereinafter, these will be specifically listed.

Examples of the ethylenically unsaturated aromatic compounds include diisopropenyl benzene, styrene, α-methylstyrene, o-methylstyrene, m-methylstyrene, p-methylstyrene, p-tert-butylstyrene, o-chlorostyrene, m-chlorostyrene, p-chlorostyene, 1,1-diphenylethylene, p-methoxystyrene, N,N-dimethyl-p-aminostyrene, N,N-diethyl-p-aminostyrene, ethylenically unsaturated pyridine and ethylenically unsaturated imidazole.

Examples of the carboxyl group-containing unsaturated compounds include (meth) acrylic acid, crotonic acid, maleic acid, fumaric acid and itaconic acid.
Examples of the monofunctional (meth)acrylates include alkyl(meth)acrylates such as methyl(meth)acrylate, ethyl(meth)acrylate, propyl(meth)acrylate, isopropyl(meth)acrylate, butyl(meth)acrylate, isobutyl(meth)acrylate, tert-butyl(meth)acrylate, pentyl(meth)acrylate, amyl(meth)acrylate, isoamyl(meth)acrylate, hexyl(meth)acrylate, heptyl(meth)acrylate, octyl(meth)acrylate, isooctyl (meth)acrylate, 2-ethylhexyl(meth)acrylate, nonyl(meth)acrylate, decyl(meth)acrylate, isodecyl(meth)acrylate, undecyl(meth)acrylate, dodecyl(meth)acrylate, lauryl(meth)acrylate, stearyl(meth)acrylate, and isostearyl(meth)acrylate; fluoroalkyl(meth)acrylatessuch as trifluoroethyl(meth)acrylate, tetrafluoropropyl(meth)acrylate, hexafluoroisopropyl(meth)acrylate, octafluoropentyl(meth)acrylate and heptadecafluorodecyl(meth)acrylate; hydroxyalkyl(meth)acrylates such as hydroxyethyl (meth) acrylate, hydroxypropyl(meth)acrylate, and hydroxybutyl(meth)acrylate; phenoxyalkyl(meth)acrylates such as phenoxyethyl(meth)acrylate, and 2-hydroxy-3-phenoxypropyl(meth)acrylate; alkoxyalkyl(meth)acrylates such as methoxyethyl(meth)acrylate, ethoxyethyl(meth)acrylate, propoxyethyl(meth)acrylate, butoxyethyl(meth)acrylate, and methoxybutyl(meth)acrylate; polyethylene glycol(meth)acrylates such as polyethylene glycol mono(meth)acrylate, ethoxy diethylene glycol(meth)acrylate, methoxy polyethylene glycol(meth)acrylate, phenoxy polyethylene glycol(meth)acrylate and nonylphenoxy polyethylene glycol(meth)acrylate; polypropylene glycol(meth)acrylates such as polypropylene glycol mono(meth)acrylate, methoxy polypropylene glycol(meth)acrylate, ethoxy polypropylene glycol(meth)acrylate and nonylphenoxy polypropylene glycol(meth)acrylate; cycloalkyl(meth)acrylates such as cyclohexyl(meth)acrylate, 4-butylcyclohexyl(meth)acrylate, dicyclopentanyl(meth)acrylate, dicyclopentenyl(meth)acrylate, dicyclopentadienyl(meth)acrylate, bornyl(meth)acrylate, isobornyl(meth)acrylate, and tricyclodecanyl(meth)acrylate; benzyl(meth)acrylate; and tetrahydrofurfuryl(meth)acrylate.

Examples of the di(meth)acrylates include ethylene glycol di(meth)acrylate, diethylene glycol di(meth)acrylate, triethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, 1,3-propane diol di(meth)acrylate, 1,4-butane diol di(meth)acrylate, 1,6-hexane diol di(meth)acrylate, 1,9-nonane diol di(meth)acrylate, hydroxyl pivalic acid ester neopentyl glycol di(meth)acrylate, bisphenol A di(meth)acrylate, 2,2-bis(4-(meth)acryloyloxyethoxyphenyl)propane, 2,2-bis(4-(meth)acryloyloxydiethoxyphenyl)propane, trimethylol propane di (meth) acrylate, tricyclodecane dimethanol diacrylate, and bis(2-(meth)acryloyloxyethyl)hydroxyethyl-isocyanurate.

Examples of the multifunctional (meth)acrylates include trimethylol propane tri(meth)acrylate, pentaerythritol tri(meth)acrylate, pentaerythritol tetra(meth)acrylate, dipentaerythritol tetra(meth)acrylate, dipentaerythritol penta(meth)acrylate, dipentaerythritol hexa(meth)acrylate, trimethylol propane trioxyethyl(meth)acrylate and tris(2-hydroxyethyl)isocyanurate tri(meth)acrylate.

Examples of the epoxy poly(meth)acrylates include those obtained by reacting a compound having two or more epoxy groups in a molecule, e.g., a bisphenol A type epoxy resin, with (meth)acrylic acid or (meth)acrylate having a hydroxyl group.

Examples of the urethane poly(meth)acrylates include a urethane di(meth)acrylate obtained by reacting a diisocyanate such as 1, 6-hexamethylene diisocyanate, isophorone diisocyanate, and dicyclohexyl methane diisocyanate with a (meth)acrylate having a hydroxyl group, such as 2-hydroxyethyl(meth)acrylate; a urethane hexa(meth)acrylate obtained by reacting 1,6-hexamethylene diisocyanate with pentaerythritol tri(meth)acrylate; and a polyurethane di (meth) acrylate obtained by reacting 2-hydroxyethyl(meth)acrylate with a urethanization reaction product of dicyclomethane diisocyanate and poly(a repeating unit n = 6 to 15) tetramethylene glycol.

Examples of the polyester poly(meth)acrylates include polyester (meth)acrylate obtained by reacting trimethylolpropane, succinic acid and (meth)acrylic acid; and polyester (meth) acrylate obtained by reacting trimethylol propane, ethylene glycol, succinic acid and (meth)acrylic acid.

The monomers having an ethylenically unsaturated bond as described above can be used singly or in combination of two or more kinds.
Further, a solvent may be contained as another component. The incorporation of a solvent can aid the dispersion of individual components of the curable composition.

Specific examples of the solvent used in the production of the curable composition of the present invention include esters such as ethyl acetate, butyl acetate, and isopropyl acetate; ketones such as acetone, methyl ethyl ketone, methyl isobutyl ketone, and cyclohexanone; cyclic ethers such as tetrahydrofuran and dioxane; amides such as N,N-dimethylformamide; aromatic hydrocarbons such as toluene; halogenated hydrocarbons such as dichloromethane; ethylene glycols such as ethylene glycol, ethylene glycol methyl ether, ethylene glycol mono-n-propyl ether, ethylene glycol monomethyl ether acetate, diethylene glycol, diethylene glycol monomethyl ether, diethylene glycol monoethyl ether, and diethylene glycol monoethyl ether acetate; propylene glycols such as propylene glycol, propylene glycol methyl ether, propylene glycol ethyl ether, propylene glycol butyl ether, propylene glycol propyl ether, propylene glycol monomethyl ether acetate, dipropylene glycol, dipropylene glycol monomethyl ether, dipropylene glycol monoethyl ether, and dipropylene glycol monomethyl ether acetate; and acetonitrile. Preferable examples include ethyl acetate, methyl ethyl ketone, cyclohexanone, toluene, dichloromethane, diethylene glycol monomethyl ether, and propylene glycol monomethyl ether acetate.

The viscosity of the curable composition of the present invention depends on the structure of the aromatic group-containing (meth)acrylate compound (1) and the amount of a solvent incorporated into the curable composition, but, at 25°C, is preferably from 10 mPa·s to 10,000 mPa·s, more preferably from 50 mPa·s to 5,000 mPa·s, most preferably from 100 mPa·s to 2,000 mPa·s.

When the viscosity of the curable composition is higher than 10,000 mPa·s, the handling characteristics are poor and the workability is inferior. The measurement conditions of the viscosity is as described in the later-described Example.

### Production Method of Curable Composition

The curable composition of the present invention can be prepared by blending the aromatic group-containing (meth)acrylate compound of the present invention, the polymerization initiator and optionally other components than the above solvent, through mixing the components under room temperature or heating condition by the use of a mixing machine such as a mixer, a ball mill and a triple roll, or through dissolving the components by further adding a solvent or the like as a diluent.

The solvents can be used singly or in combination of two or more kinds.
The amount used of the solvent is not particularly limited, but is usually 50 to 200 parts by mass, preferably 50 to 100 parts by mass, based on 100 parts by mass of the total of the components, excluding the solvent, of the curable composition.

### Production Method of Cured Article

The cured article of the present invention can be obtained, for example, by applying the curable composition on a substrate so as to form a coating film and then by applying an active energy ray or heating so as to cure the film. The curing may be carried out both by applying an active energy ray and by heating.

As a method for applying the curable composition, for example, there can be mentioned a coating with a bar coater, an applicator, a die coater, a spin coater, a spray coater, a curtain coater, a roll coater or the like; coating by screen printing; and coating by dipping.

The amount of the curable composition of the present invention to be applied on the substrate is not particularly limited and can be controlled appropriately in accordance with a purpose. The amount is preferably such that a coating film obtained after coating and drying and curing treatment by an active energy ray will have a film thickness of 1 to 500 µm, more preferably 5 to 300 µm, as a thickness for the evaluation of the properties of the cured article.

The active energy ray used for curing is preferably an electron ray and a light with a wavelength ranging from ultraviolet region to infrared region. Regarding a light source that can be used, for example, if the active energy ray is ultraviolet, an ultra-high pressure mercury light source or a metal halide light source can be used; and if the active energy ray is a visible light, a metal halide light source or a halogen light source can be used; and if the active energy ray is infrared ray, a halogen light source can be used. In addition thereto, further examples of the light source include laser and LED. The application amount of the active energy ray is determined appropriately in accordance with the type of a light source, the thickness of a coating film, and the like.

The application amount of the active energy ray is determined appropriately in accordance with the type of a light source, the thickness of a coating film and the like, but preferably, is determined appropriately so that the reactivity rate of the ethylenically unsaturated group of the aromatic group-containing (meth) acrylate compound (1) will become 80% or more, more preferably 90% or more. The reactivity rate is calculated using infrared absorption spectrum from the variation between the absorption peak strengths of the ethylenically unsaturated group before reacted and of the ethylenically unsaturated group after reacted.

The curing by the application of an active energy ray may be followed by, as needed, further curing through heating treatment or annealing treatment. At this time, the heating temperature is preferably 80 to 200°C. The heating time is preferably 10 to 60 minutes.

For the curing of the curable composition of the present invention, when heat treatment is carried out to cause the thermal polymerization, the heating temperature is preferably 80 to 200°C, more preferably 100 to 150°C. When the heating temperature is lower than 80 °C, the heating time needs to be longer, which tends to be uneconomical, and when the heating time is higher than 200°C, in addition to higher energy cost, more time is needed to raise temperature by heating and to lower temperature, which is uneconomical.

The heating time is determined appropriately in accordance with the heating temperature, the thickness of a coating film and the like, but preferably, is determined appropriately so that the reactivity rate of the ethylenically unsaturated group of the aromatic group-containing (meth)acrylate compound (1) will become 80% or more, more preferably 90% or more. The reactivity rate is calculated using infrared absorption spectrum from the variation between the absorption peak strengths of the ethylenically unsaturated group before reacted and of the ethylenically unsaturated group after reacted.

### Cured Article

The cured article obtained by the above method preferably has an refractive index of 1.55 or higher, more preferably 1.56 or higher, still more preferably 1.57 or higher, most preferably 1.58 or higher.

When the refractive index of the cured article is lower than 1. 55, an optical lens or the like has a thickened central portion, and thus may impair its lightness, a feature of a plastic. The refractive index of the cured article of the present invention is preferably 1.65 or lower, more preferably 1.64 or lower. When the refractive index of the cured article is higher than 1.65, the light surface reflection and light scattering loss may reduce the transparency.

The cured article obtained by the above method preferably has a glass transition temperature of 80°C or higher, more preferably 90°C or higher, most preferably 100°C or higher.

When the glass transition temperature of the cured article is lower than 80°C, the heat resistance is inferior and coloration and warpage may occur. Herein, the glass transition temperature is a temperature obtained by the method indicated in the later-described of Example.

The cured article of the present invention preferably has a glass transition temperature of 200°C or lower, more preferably 190°C or lower. When the glass transition temperature of the cured article is higher than 200°C, concern may arise in the processability.

### Examples

Hereinafter, the present invention will be descried in detail with reference to Example and Comparative Example, but in no way is the present invention limited by the descriptions provided therein.

### [Synthesis of Aromatic Group-Containing (Meth)acrylate Compound (1)]

### (Example 1) Aromatic group-containing methacrylate compound (A-1)

In a reaction vessel, 300 parts by mass of toluene (manufactured by Junsei Chemical Co., Ltd.), 96 parts by mass of glycerol dimethacrylate (manufactured by Shin-Nakamura Chemical Co., Ltd.), and 43 parts by mass of triethylamine (manufactured by Tokyo Chemical Industry Co., Ltd.) were introduced and stirred. Then, under cooling with ice, 80 parts by mass of 1-naphtoyl chloride (manufactured by Tokyo Chemical Industry Co., Ltd.) was gradually dropped, and stirred at room temperature. 15 hours thereafter, the glycerol dimethacrylate, the ingredient, was confirmed by high performance liquid chromatography to have almost disappeared, and then pure water was added to the reaction liquid to terminate the reaction. Subsequently, the extraction using ethyl acetate was carried out, and then the ethyl acetate phase was washed two times using a saturated saline solution. The ethyl acetate phase was dried with anhydrous sodium sulfate and concentrated under reduced pressure, thereby to obtain an aromatic group-containing methacrylate compound (A-1).

The aromatic group-containing methacrylate compound (A-1) was found to have a viscosity equivalent to the viscosity of the later-mentioned solution 1, such that the difference between the viscosities was approximately within 20%. The measurement conditions are the same as those for the solution 1.
The ¹H-NMR chart of the aromatic group-containing methacrylate compound (A-1) is shown in Fig. 1. The measurement of ¹H-NMR employed AMX400 manufactured by Bruker Corporation, and the measurement was carried out in chloroform-d. The assignment of the ¹H-NMR chart is indicated below. From the result of the ¹H-NMR, the aromatic group-containing methacrylate compound (A-1) was found to have a molecular structure represented by the following formula (6).

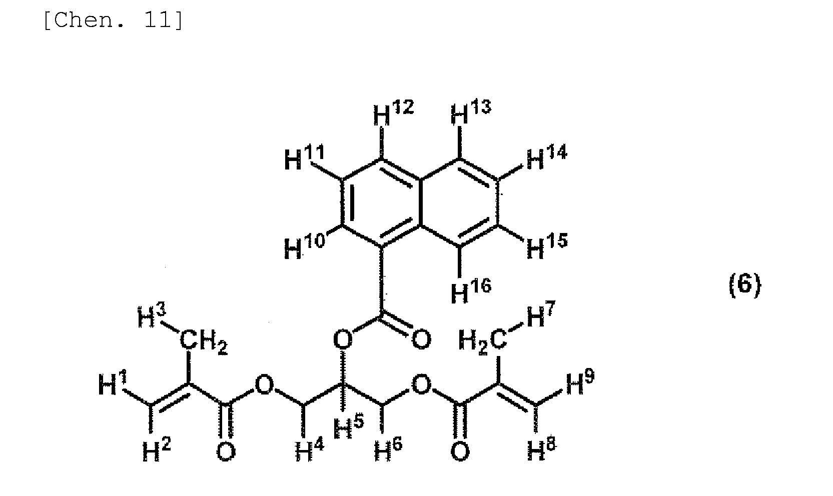

1.93 ppm: H³, H⁷
4.43-4.58 ppm: H⁴, H⁶
5.59 ppm: H¹, H⁹
5.68 ppm: H⁵
6.14 ppm: H², H⁸
7.47-7.59 ppm: H¹¹, H¹⁴, H¹⁵
7.88 ppm: H¹³
8.02 ppm: H¹²
8.15 ppm: H¹⁰
8.84 ppm: H¹⁶

### (Example 2) Aromatic group-containing (meth)acrylate compound (A-2)

The same procedure was carried out as in Example 1, except that 90 parts by mass of glycerol acrylate methacrylate (manufactured by Shin-Nakamura Chemical, Co., Ltd.) was used instead of glycerol dimethacrylate, thereby to obtain an aromatic group-containing (meth)acrylate compound (A-2).

The aromatic group-containing (meth)acrylate compound (A-2) was found to have a viscosity equivalent to the viscosity of the later-mentioned solution 2, such that the difference between the viscosities was approximately within 20%. The measurement conditions are the same as those for the solution 2.

### (Example 3) Aromatic group-containing methacrylate compound (A-3)

The same procedure was carried out as in Example 1, except that 80 parts by mass of 2-naphtoyl chloride (manufactured by Tokyo Chemical Industry Co., Ltd.) was used instead of 1-naphtoyl chloride, thereby to obtain an aromatic group-containing methacrylate compound (A-3).

The aromatic group-containing methacrylate compound (A-3) was found to have a viscosity equivalent to the viscosity of the later-mentioned solution 3, such that the difference between the viscosities was approximately within 20%. The measurement conditions are the same as those for the solution 3.

### (Example 4) Aromatic group-containing methacrylate compound (A-4)

The same procedure was carried out as in Example 1, except that 91 parts of 4-phenylbenzoyl chloride (manufactured by Tokyo Chemical Industry Co., Ltd.) was used instead of 1-naphtoyl chloride, thereby to obtain an aromatic group-containing methacrylate compound (A-4).

The aromatic group-containing methacrylate compound (A-4) was found to have a viscosity equivalent to the viscosity of the later-mentioned solution 4, such that the difference between the viscosities was approximately within 20%. The measurement conditions are the same as those for the solution 4.

### (Example 5) Aromatic group-containing methacrylate compound (A-5)

Under nitrogen atmosphere, in a reaction vessel, 1740 parts by mass of dichloromethane (manufactured by Junsei Chemical Co., Ltd.), 100 parts by mass of glycerol dimethacrylate (manufactured by Shin-Nakamura Chemical Co., Ltd.), 91 parts by mass of 4-phenyl benzoic acid (manufactured by Tokyo Chemical Industry Co., Ltd.), and 29 parts by mass of N,N-dimethyl-4-aminopyridine (manufactured by Tokyo Chemical Industry Co., Ltd.) were introduced and stirred. Then, under cooling with ice, 46 parts by mass of 1-ethyl-3-(3-dimethylaminopropyl)carbodiimide (manufactured by Tokyo Chemical Industry Co., Ltd.) was added over 10 minutes, and 25 minutes thereafter, 46 parts by mass of 1-ethyl-3-(3-dimethylaminopropyl)carbodiimide was added over 8 minutes and stirred at room temperature. 12 hours thereafter, the glycerol dimethacrylate, the ingredient, was confirmed by high performance liquid chromatography to have almost disappeared, and then 0.5 N hydrochloric acid aqueous solution was added to the reaction liquid to terminate the reaction. Subsequently, the extraction using dichloromethane was carried out, and then the dichloromethane phase was washed using a saturated sodium bicarbonate water and pure water. The dichloromethane phase was dried with anhydrous sodium sulfate and concentrated under reduced pressure, thereby to obtain an aromatic group-containing methacrylate compound (A-5).

The ¹H-NMR chart of the aromatic group-containing methacrylate compound (A-5) is shown in Fig. 2. The measurement of ¹H-NMR employed AMX400 manufactured by Bruker Corporation, and the measurement was carried out in chloroform-d. The assignment of the ¹H-NMR chart is indicated below. From the result of the ¹H-NMR, the aromatic group-containing methacrylate compound (A-5) was found to have a molecular structure represented by the following formula (7).

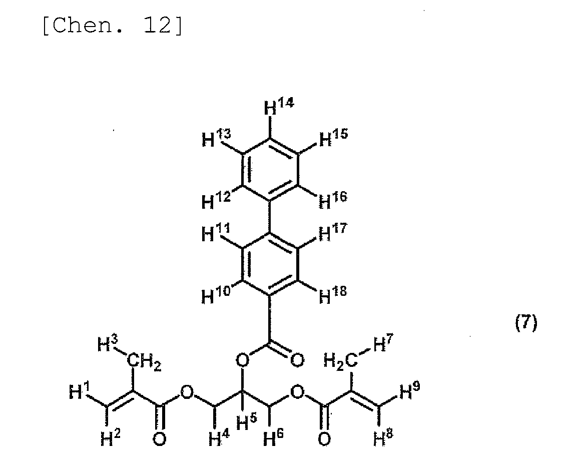

1.93 ppm: H³, H⁷
4.41-4.52 ppm: H⁴, H⁶
5.57-5.64 ppm: H¹, H⁵, H⁹
6.11 ppm: H², H⁸
7.38-7.47 ppm: H¹³, H¹⁴, H¹⁵
7.59-7.66 ppm: H¹¹, H¹², H¹⁶, H¹⁷
8.06-8.09 ppm: H¹⁰, H¹⁸

### (Comparative Example 1) (Meth)acrylate compound (B-1)

In a reaction vessel, 150 parts of glycerol acrylate methacrylate (manufactured by Shin-Nakamura Chemical Co., Ltd.), and 2 parts of dibutyltin dilaurirate (manufactured by Tokyo Chemical Industry Co., Ltd.) were introduced and stirred. Then, 99 parts of 2-acryloyloxyethyl isocyanate (manufactured by Showa Denko K.K.; product name Karenz AOI(trade name)) was gradually dropped, and stirred at room temperature. The glycerol acrylate methacrylate, the ingredient, was confirmed by high performance liquid chromatography to have almost disappeared, and then the reaction was terminated. Subsequently, the reaction liquid was washed four times using 150 parts of hexane containing 200 ppm of 2,6-di-tert-butyl-4-methylphenol (BHT, manufactured by Junsei Chemical Co., Ltd.), thereby to obtain a (meth)acrylate compound (B-1).

The (meth)acrylate compound (B-1) was found to have a viscosity equivalent to the viscosity of the later-mentioned solution 5, such that the difference between the viscosities was approximately within 20%. The measurement conditions are the same as for the solution 5.

### [Preparation of Curable Composition]

### (Example 6) Curable Composition (solution 1)

In a vessel which was shielded from ultraviolet, 100 parts by mass of the aromatic group-containing methacrylate compound (A-1) prepared in Example 1, 1 part by mass of ethyl(2,4,6-trimethylbenzoyl)phenylphosphinate (manufactured by BASF Japan Co., Ltd.; product name: Lucirin TPO-L) was blended as a photopolymerization initiator, and stirred and mixed at room temperature and homogenously dissolved, thereby to obtain a curable composition (solution 1).

### (Example 7) Curable Composition (solution 2)

The same procedure was carried out as in Example 6, except that the aromatic group-containing (meth)acrylate compound (A-2) was used instead of the aromatic group-containing methacrylate compound (A-1), thereby to obtain a curable composition (solution 2).

### (Example 8) Curable Composition (solution 3)

The same procedure was carried out as in Example 6, except that the aromatic group-containing methacrylate compound (A-3) was used instead of the aromatic group-containing methacrylate compound (A-1), thereby to obtain a curable composition (solution 3).

### (Example 9) Curable Composition (solution 4)

The same procedure was carried out as in Example 6, except that the aromatic group-containing methacrylate compound (A-4) was used instead of the aromatic group-containing methacrylate compound (A-1), thereby to obtain a curable composition (solution 4).

### (Comparative Example 2) Comparative Curable Composition (solution 5)

The same procedure was carried out as in Example 6, except that the (meth)acrylate compound (B-1) was used instead of the aromatic group-containing methacrylate compound (A-1), thereby to obtain a comparative curable composition (solution 5).

### (Comparative Example 3) Comparative Curable Composition (solution 6)

The same procedure was carried out as in Example 6, except that o-phenylphenoxy ethylacrylate (manufactured by TOAGOSEI Co., Ltd.) was used instead of the aromatic group-containing methacrylate compound (A-1), thereby to obtain a comparative curable composition (solution 6).

### (Comparative Example 4) Comparative Curable Composition (solution 7)

The same procedure was carried out as in Example 6, except that a bisarylfluorene skeleton compound (manufactured by Osaka Gas Chemicals, Co., Ltd.; product name: OGSOL EA-0200) was used instead of the aromatic group-containing methacrylate compound (A-1), thereby to obtain a comparative curable composition (solution 7).

### [Production of Cured Article]

The curable compositions (solutions 1 to 4) prepared in Examples 6 to 9 and comparative curable compositions (solutions 5 to 7) prepared in Comparative Examples 2 to 4 were each separately applied on a glass substrate (50 mm x 50 mm) to form a film such that a cured article would have a thickness of 200 µm. Subsequently, the coating film was cured using an irradiation device equipped with an ultra-high-pressure mercury lamp at 4 J/cm², thereby to obtain a cured article.

### [Property Evaluation Method]

### (1) Viscosity

The viscosity of the curable compositions (solutions 1 to 7) was measured using a B-type viscometer DV-II+Pro (manufactured by Brookfield Engineering Laboratories, Inc.), a rotor No. 42, a rotation number of 1 to 7 rpm, at a measurement temperature of 25°C. The results are set forth in Table 1.

The curable composition with a moderately lower viscosity can be said to have good handling characteristics.

### (2) Refractive index

The refractive index of the cured article obtained was measured at a measurement temperature of 25°C, using a Multi-Wavelength Abbe Refractometer DR-M2 (manufactured by Atago Co., Ltd.). The measurement wavelength was 589 nm. The results are set forth in Table 1.

### (3) All Light Transmittance

All light transmittance of the cured article obtained was measured, using a haze meter, COH400 (manufactured by Nippon Denshoku Industries, Co., Ltd.). The results are set forth in Table 1.

### (4) Glass Transition Temperature (Tg)

The cured article obtained was processed into the size of 30 mm in length, 5 mm in width and 200 µm in thickness, which was subjected to the measurement using DMS6100 (manufactured by SEIKO Electronics Industries Ltd.), at a tensile mode, at temperatures ranging from 20°C to 300°C, by elevating temperature at a rate of 2°C/min, at a frequency of 1 Hz, to give a tan 5 value. The peak temperature of the tan δ value was defined as a glass transition temperature. The results are set forth in Table 1.

The cured article with a higher glass transition temperature can be said to have good heat resistance.

### (5) Pencil Hardness

In accordance with JIS-K5600, the cured article was scratched with a UNI (trade name) produced by Mitsubishi Pencil Co., Ltd. such that the angle formed by the pencil and the cured article would be 45 degrees, and the hardest pencil causing no scratch was measured and the hardness was defined as the pencil hardness, which is set forth in Table 1.

[Table 1]

**Table 1**

| | Ex. 6 | Ex. 7 | Ex. 8 | Ex. 9 | Com. Ex. 2 | Com. Ex. 3 | Com. Ex. 4 |
|---|---|---|---|---|---|---|---|
| Viscosity (mPa·s) | 458 | 403 | 1,523 | 305 | 781 | 129 | >100,000 |
| Refractive index n_{D}²⁵ | 1.5847 | 1.5895 | 1.5889 | 1.5855 | 1.5163 | 1.6096 | 1.6260 |
| All light transmittance (%) | 92 | 92 | 92 | 92 | 92 | 91 | - |
| Glass transition temperature Tg(°C) | 150 | 109 | 129 | 139 | >250 | <30 | 211 |
| Pencil hardness | 5H | 3H | 4H | 4H | 8H | HB | - |

Table 1 shows that the compositions of Examples 6 to 9 of the present invention have a low viscosity and good handling characteristics. Further, the cured articles have a refractive index of not lower than 1.55 and have good all light transmittance, being able to be suitably used for a material for an optical lens and the like. Moreover, the cured articles have a glass transition temperature of not lower than 80 °C, and thus have good heat resistance. Furthermore, the cured articles have a pencil hardness of 3H or more, being able to be suitably used for a coating material, too.

In comparison therewith, in Comparative Example 2, containing the (meth) acrylate compound (B-1), the viscosity of the composition, and the transparency, heat resistance and pencil hardness of the cured article are good, but the refractive index of the cured article is moderate and is inferior as an optical material.

In Comparative Example 3, containing o-phenylphenoxy ethylacrylate, the viscosity of the composition, and the refractive index and transparency of the cured article are good, but because of the monofunction, the heat resistance of the cured article is inferior and the pencil hardness is also lower.

In Comparative Example 4, containing bisarylfluorene skeleton compound, the refractive index and heat resistance of the cured article are good, but the viscosity of the composition is extremely high and the handling characteristics are inferior.

### Industrial Applicability

As described above, the aromatic group-containing (meth)acrylate compound and the curable composition of the present invention have low viscosity and thus have superior handling characteristics, and therefore can improve the workability.
Moreover, the cured article obtained by curing the curable composition containing the aromatic group-containing (meth)acrylate compound has a high refractive index and good transparency and heat resistance, and thus is suitable as an optical material. Examples of the optical material include a transparent substrate, an optical lens, a optical disk substrate, a plastic substrate for a liquid crystal display, a substrate for a color filter, a plastic substrate for an organic EL display element, a solar cell substrate, a touch panel, an optical element, an optical waveguide, a LED sealant.

Furthermore, the cured article obtained by curing the curable composition containing the aromatic group-containing (meth) acrylate compound has an excellent pencil hardness, too, and therefore is suitable as a coating material, too. Examples of the coating material include a liquid crystal television, a personal computer, a display for a mobile phone, and a touch panel.

## Claims

1. An aromatic group-containing (meth)acrylate compound represented by the following general formula (1): 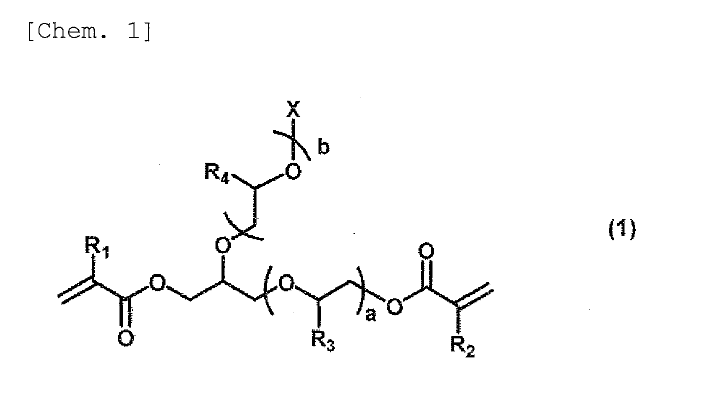 wherein R₁, R₂, R₃ and R₄ are each independently a hydrogen atom or a methyl group; X is an organic group having an aromatic group and 6 to 30 carbon atoms; and a and b are each independently an integer of 0 to 3.

2. An aromatic group-containing (meth) acrylate compound represented by the following general formula (2): 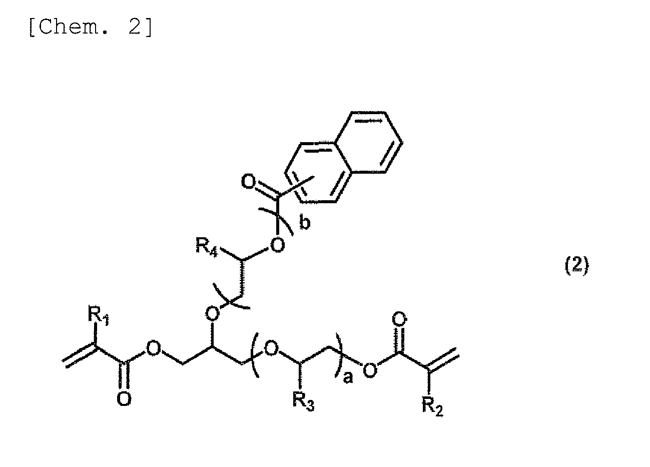 wherein R₁, R₂, R₃ and R₄ are each independently a hydrogen atom or a methyl group; and a and b are each independently an integer of 0 to 3.

3. An aromatic group-containing (meth)acrylate compound represented by the following general formula (3): 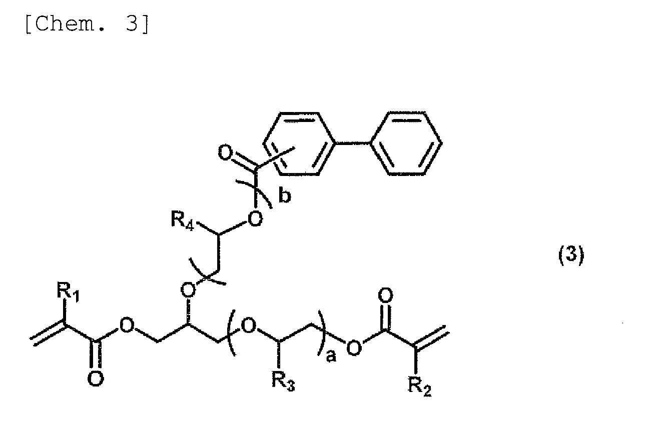 wherein R₁, R₂, R₃ and R₄ are each independently a hydrogen atom or a methyl group; and a and b are each independently an integer of 0 to 3.

4. A curable composition comprising the aromatic group-containing (meth)acrylate compound as claimed in any one of claims 1 to 3 and a polymerization initiator.

5. The curable composition as claimed in claim 4, which comprises 0.1 to 5 parts by mass of the polymerization initiator based on 100 parts by mass of the aromatic group-containing (meth)acrylate compound.

6. The curable composition as claimed in claim 4, which further comprises a urethane oligomer and/or a reactive monomer.

7. The curable composition as claimed in claim 6, which comprises 0.1 to 5 parts by mass of the polymerization initiator based on 100 parts by mass of the total of the aromatic group-containing (meth)acrylate compound and the urethane oligomer and/or the reactive monomer.

8. The curable composition as claimed in any one of claims 4 to 7, which has a viscosity at 25°C of from 10 mPa·s to 10,000 mPa·s.

9. A cured article which is obtained by curing the curable composition as claimed in any one of claims 4 to 8 and which has a refractive index of from 1.55 to 1.65.

10. A cured article which is obtained by curing the curable composition as claimed in any one of claims 4 to 8 and which has a glass transition temperature of 80°C to 200°C.

11. A coating material which is obtained by curing the curable composition as claimed in any one of claims 4 to 8.

12. An optical material which is obtained by curing the curable composition as claimed in any one of claims 4 to 8.

13. A lens which is obtained by curing the curable composition as claimed in any one of claims 4 to 8.
